# EUROPÄISCHE PATENTSCHRIFT

(11) **EP 1 901 605 B1**
(45) Veröffentlichungstag und Bekanntmachung des Hinweises auf die Patenterteilung: **14.05.2014**
(21) Anmeldenummer: 06754641.6
(22) Anmeldetag: 30.06.2006
(51) Int. Cl.: A01N 1/02

(54) **LAGERMEDIUM FÜR ZELLEN**
STORAGE MEDIUM FOR CELLS
MILIEU DE CONSERVATION POUR CELLULES

(30) Priorität: 01.07.2005 DE 102005031532
(43) Veröffentlichungstag der Anmeldung: 26.03.2008
(73) Patentinhaber: Cytonet GmbH & Co. KG, 69469 Weinheim/Bergstrasse (DE)
(72) Erfinder: ARSENIEV, Lubomir, 30625 Hannover (DE); ALEXANDROVA, Krassimira, 30625 Hannover (DE); BARTHOLD, Marc, 30171 Hannover (DE); GRIESEL, Carsten, Cary, NC 27513 (US); HEUFT, Hans-Gerd, 30952 Ronnenberg (DE); KAFERT-KASTING, Sabine, 30173 Hannover (DE); PRIESNER, Christoph, 30161 Hannover (DE)
(74) Vertreter: Schwahn, Hartmut
(86) Internationale Anmeldenummer: PCT/EP2006/006401
(87) Internationale Veröffentlichungsnummer: WO 2007/003382

(56) Entgegenhaltungen:
- EP-A1- 1 149 900
- MARSH D C ET AL: "HYPOTHERMIC PRESERVATION OF HEPATOCYTES II. IMPORTANCE OF CA2 AND AMINO ACIDS" CRYOBIOLOGY, ACADEMIC PRESS INC, US, Bd. 27, Nr. 1, Februar 1990 (1990-02), Seiten 1-8, XP000973919 ISSN: 0011-2240

## Beschreibung

Die Erfindung betrifft Verfahren zur Lagerung von insbesondere humanen Leberzellen, Zellmedien für die Lagerung von Leberzellen, sowie die Verwendung solcher Zellmedien zur Lagerung der Zellen, wobei die Vitalität der Zellen während der Lagerung erhalten bleibt.

Die Kryokonservierung stellt eine häufig verwendete Methode zur Lagerung lebender Zellen über längere Zeiträume dar. Die Kryokonservierungstechniken wurden im Laufe des letzten Jahrzehnts stetig weiterentwickelt und verbessert. Es besteht ein Bedürfnis Zellen auch kurzfristig zu lagern, zum Beispiel unmittelbar nach Isolierung aus einem Organ, vor dem Einfrieren oder nach oder während dem Auftauen der Zellen nach Kryokonservierung. Oft ist es erforderlich, dass die Zellen nach dieser Lagerung einem Organismus, insbesondere einem Menschen, wieder unmittelbar zugeführt werden sollen. Dieses Zuführen der Zellen kann beispielsweise durch eine intravenöse Applikation der Zellen erfolgen. Die Applikation von, besonders mittels Kryokonservierung, gelagerten Zellen in einen Menschen kann vor allem als klinische, insbesondere als therapeutische Anwendung genutzt werden. So können beispielsweise humane Leberzelltransplantate zur therapeutischen Anwendung intravenös appliziert werden. Jedoch stellt der Einfrierprozess und der Wiederauftauprozess und die vorausgehende beziehungsweise direkt anschließende *in vitro* Lagerung in Bezug auf die Vitalität und die Lebendzellzahl der zu lagernden Zellen besonders bei der klinischen Anwendung weiterhin ein Problem dar.

Der vorliegenden Erfindung liegt das technische Problem zugrunde, ein einfach herstellbares und möglichst preiswertes Zellmedium für die Lagerung von Leberzellen bereitzustellen. Die Zellen sollen bevorzugt im Zusammenhang mit der Kryokonservierung vor dem Einfrieren und/oder nach dem Auftauen gelagert werden. Der vorliegenden Erfindung liegt daher auch das technische Problem zugrunde, ein Zellmedium bereitzustellen, dass ein kurzzeitiges Lagern von tierischen Zellen, bevorzugt von humanen Zellen, besonders bevorzugt von Leberzellen oder Leberzelltransplantaten, bei einem möglichst geringen Verlust der Vitalität und der Lebendzellzahl der Zellen erlaubt und somit eine Alternative zu bekannten Zellmedien darstellt.

Im Zusammenhang mit der Kryokonservierung sind Zellmedien zum Lagern und Wiederauftauen bekannt. Sie enthalten jedoch Bestandteile wie 5 bis 20 % fötales Kalbserum (FCS), die für eine Applikation, insbesondere eine intravenöse Applikation, in den Menschen gesetzlich nicht zugelassen sind, oder sogar ungeeignet sind.

Die EP 1 149 900 A1 offenbart ein Verfahren zur Lagerung von Leberzellen bei niedrigen Temperaturen. Dazu wird eine Lösung eingesetzt, die Natriumlactobionat, Magnesiumsulfat, Kaliumphosphat, Raffinose und Glutathion enthält. Die Publikation Marsh D.C. et al. "Hypothermic Preservation of Hepatocytes, II. Importance of Ca2+ and Amino Acids", Cryobiology, Academic Press Inc. US, 27 (1), February 1990:1-8, beschreibt Lagermedien und Minimalanforderungen für die hypotherme Lagerung von Hepatozyten. Ein daraus bekanntes Zellkulturmedium weist Natriumchlorid, Kaliumchlorid, Kalium/Natriumhydrogenphosphat, Natriumhydrogencarbonat und Polyethylenglycol (PEG) sowie einen Zusatz von Kalzium- und Magnesiumionen sowie Aminosäuren und Substrat wie Glucose auf.

Der vorliegenden Erfindung liegt daher weiter das technische Problem zugrunde, ein Medium zur Verfügung zu stellen, dass bei einem geringen Verlust der Vitalität und der Lebendzellzahl von Leberzellen eine risikofreie Applikation der Zellen in ein Tier, insbesondere einen Menschen, erlaubt.

So lässt sich Custodiol^{®} als Zellmedium zum Beispiel für die Lagerung besonders von aufgetauten, ursprünglich kryokonservierten Zellen wie Leberzellen erfolgreich einsetzen; es wurde als Konservierungslösung für die Organtransplantation von Ganzorganen entwickelt. Custodiol^{®} ist jedoch für eine intravenöse Applikation beim Menschen gesetzlich nicht zugelassen. Der Erfindung liegt daher auch das technische Problem zugrunde, ein Zellmedium für die kurzzeitige Lagerung von Zellen, insbesondere im Zusammenhang mit der Kryokonservierung, zur Verfügung zu stellen, das für eine intravenöse Applikation beim Menschen zugelassen ist, und daher die Zellen beispielsweise nach dem Auftauen ohne aufwändige und die Lebendzellzahl vermindernde Waschschritte einem Menschen in diesem Zellmedium intravenös appliziert werden können.

Die vorliegende Erfindung löst das ihr zugrundeliegende technische Problem im Wesentlichen durch die Bereitstellung eines Verfahrens für die kurzzeitige Lagerung tierischer oder humaner Leberzellen gemäß der Patentansprüche. Es wird ein Verfahren bereitgestellt, wobei die Zellen in einem flüssigen Zellmedium, das Salzlösung, enthaltend Natriumchlorid, Natriumglukonat, Natriumacetat, Kaliumchlorid, Magnesiumchlorid und Natriumcitrat, sowie Glucose und darüber hinaus Serumalbumin und/oder Blutplasma enthält, aufgenommen werden und in diesem Zellmedium gelagert werden.

In bevorzugten Ausführungsformen sind die Leberzellen Säugetier-Zellen. Es kann sich beispielsweise um porcine, bovine oder murine Zellen handeln. Besonders bevorzugt handelt es sich um humane Zellen. Unter Zellen sind erfindungsgemäß auch Zellsuspensionen und Zelltransplantate gemeint. Die Zellen können von einem Typ sein, es kann aber auch eine Zusammensetzung beziehungsweise Co-Kultur verschiedener Zelltypen eingesetzt werden.

Erfindungsgemäß bevorzugt handelt es sich bei den tierischen Zellen um Zellen zur therapeutischen Applikation. In einer weiteren bevorzugten Ausführungsform handelt es sich bei den tierischen Zellen um Zellen zur *in vitro* Kultivierung.

Im Zusammenhang mit dieser Erfindung wird unter Leberzellen eine Mischung von Zellen vorwiegend bestehend aus Hepatozyten verstanden. Sie können jedoch auch verschiedene Mengenanteile an Lymphozyten, Endothelzellen und weiteren nicht-parenchymalen Zellgruppen enthalten.

Das Auftauen und/oder Lagern von Thrombozyten, auch Blutplättchen genannt, ist von der Erfindung ausgenommen. Thrombozyten sind keine tierischen Zellen im Sinne der Erfindung.

Im Zusammenhang mit dieser Erfindung wird unter kurzzeitiger Lagerung eine *in vitro* Lagerung in einem erfindungsgemäßen Medium über einen Zeitraum bis 24 Stunden, besonders bis 10 Stunden, bevorzugt bis 5 Stunden, besonders bis 3 Stunden verstanden.

Unter Kryokonservierung wird eine meist langfristige Lagerung von Zellen unterhalb von 0 °C, insbesondere bei -20 °C bis -200 °C verstanden. Das Einfrieren erfolgt in der Regel bei 1 °C/min.

Im Zusammenhang mit dieser Erfindung wird unter einem Zellmedium, auch Medium, Auftaumedium oder Nährmedium genannt, insbesondere eine flüssige wässrige Lösung verstanden. Das Zellmedium dient der *in* vitro-Lagerung lebender Zellen unter Beibehaltung einer möglichst hohen Lebendzellzahl, also einer möglichst geringen Sterberate der Zellen, sowie einer möglichst hohen Lebensfähigkeit beziehungsweise Vitalität, dem Fachmann auch als *viability* bekannt.

Erfindungsgemäß bevorzugt sind die Bestandteile eines erfindungsgemäß eingesetzten Zellmediums gesetzlich für die Applikation, insbesondere die intravenöse Applikation, in einen tierischen Organismus, bevorzugt in einen menschlichen Organismus, zugelassen. Erfindungsgemäß können die Zellen nach der Lagerung in einem erfindungsgemäßen Medium für eine klinische Anwendung, insbesondere einer therapeutischen Applikation, oder für *in vitro* Versuche verwendet werden.

Im Zusammenhang mit dieser Erfindung wird unter einer Salzlösung eine wässrige Lösung von einem oder mehreren Salzen verstanden. Die erfindungsgemäße Salzlösung enthält Natriumchlorid, Natriumglukonat, Natriumacetat, Kaliumchlorid, Magnesiumchlorid und Natriumcitrat, wobei die Salze in Wasser gelöst sind. Das benutzte Wasser ist steriles, bevorzugt deionisiertes Wasser zur Injektion.

Eine erfindungsgemäß bevorzugte Salzlösung ist Composol^{®} beziehungsweise eine der Zusammensetzung von Composol^{®} entsprechende Lösung. Composol^{®} ist eine wässrige Lösung von Natriumchlorid, Natriumglukonat, Natriumacetat, Kaliumchlorid, Magnesiumchloridhexahydrat und Natriumcitrat. Sie enthält 173 mmol/l Natrium, 5 mmol/l Kalium, 1,5 mmol/l Magnesium, 98 mmol/l Chlorid, 10,9 mmol/l Citrat, 27 mmol/l Acetat und 23 mmol/l Glukonat, bevorzugt besteht sie daraus.

Erfindungsgemäß bevorzugt enthält die Salzlösung daher von 0,45 bis 0,60 Gew.-%, besonders bevorzugt 0,53 Gew.-%, Natriumchlorid, von 0,45 bis 0,55 Gew.-%, besonders bevorzugt 0,50 Gew.-%, Natriumglukonat, von 0,20 bis 0,25 Gew.-%, besonders bevorzugt 0,22 Gew.-%, Natriumacetat, von 0,03 bis 0,04 Gew.-%, besonders bevorzugt 0,037 Gew.-%, Kaliumchlorid, von 0,025 bis 0,035 Gew.-%, besonders bevorzugt 0,031 Gew.-%, Magnesiumchloridhexahydrat, und von 0,31 bis 0,33 Gew.-% , besonders bevorzugt 0,32 Gew.-%, Natriumcitrat, bevorzugt besteht sie daraus. Die Salzlösung hat bevorzugt einen pH-Wert von 7,0 bis 7,4, besonders bevorzugt von 7,2.

In einer weiteren Ausführungsform ist Bestandteil des Mediums eine wässrige Lösung mindestens eines hochmolekularen Zuckers. Erfindungsgemäße hochmolekulare Zucker sind beispielsweise Dextran, auch Dextranlösungen wie Perfadex, Hydroxyethylstärke (HES) und Derivate der Hydroxyethylstärke. Erfindungsgemäß bevorzugt wird als hochmolekularer Zucker Hydroxyethylstärke verwendet.

Erfindungsgemäß wird die Glucose dem Zellmedium in fester Form oder als wässrige Lösung zugegeben.

Erfindungsgemäß wird das Serumalbumin in Zellmedium gelöst oder in flüssiger Form zugegeben. Serumalbumin kann beispielsweise als fötales Kälberserum (FCS), bovines Serumalbumin (BSA) oder humanes Serumalbumin (HSA) vorliegen. Erfindungsgemäß bevorzugt ist humanes Serumalbumin, da dieses für die intravenöse Applikation im Menschen zugelassen ist.

In einer weiteren Ausführungsform ist Serumalbumin durch Blutplasma ersetzt oder ergänzt. Erfindungsgemäß bevorzugt ist humanes, bevorzugt autologes, Blutplasma. Besonders wird Plasma und/oder HSA als Kryoprotektor, bevorzugt allein oder in Verbindung mit anderen bekannten Kryoprotektoren, insbesondere DMSO, eingesetzt.

In weiteren bevorzugten Ausführungsformen enthält das erfindungsgemäß eingesetzte Medium mindestens einen weiteren Bestandteil, ausgewählt aus Aminosäuren, Hormonen, Vitaminen, Provitaminen und antiapoptotisch wirksamen Substanzen.

Die Bestandteile der Medienzusammensetzung wird der Fachmann je nach Einsatzgebiet nach seinem Fachwissen auswählen, nachdem er durch die erfindungsgemäße Lehre dazu angehalten wird, sie entsprechend der Erfindung und in den erfindungemäßen Konzentrationen einzusetzen.

Das erfindungsgemäße Verfahren wird bevorzugt mit einem vorgenannten Zellmedium mit einem pH-Wert von 6,4 bis 8, bevorzugt von 7,0 bis 7,4, insbesondere von 7,2 durchgeführt.

Bevorzugt werden die Zellen nach dem Auftauen aus der Kryokonservierung in erfindungsgemäßem Zellmedium gewaschen, vom Konservierungsmedium und dem erfindungsgemäßem Zellmedium abgetrennt und dann in frischem erfindungsgemäßem Zellmedium zur Lagerung aufgenommen. Die Abtrennung erfolgt bevorzugt durch Abzentrifugieren.

Das Verfahren wird bevorzugt bei einer Temperatur von 2 bis 40 °C durchgeführt. Das Zellmedium hat bevorzugt eine Temperatur von 2 bis 40 °C. Besonders bevorzugt wird das Verfahren bei 4 °C durchgeführt und/oder das verwendete Medium hat eine Temperatur von 4 °C. In einer weiteren bevorzugten Variante wird das Verfahren bei 10 °C durchgeführt und/oder das verwendete Medium hat eine Temperatur von 10 °C. In einer weiteren besonders bevorzugten Variante wird das Verfahren bei 25 °C durchgeführt und/oder das verwendete Medium hat eine Temperatur von 25 °C. In einer weiteren besonders bevorzugten Variante wird das Verfahren bei 37 °C durchgeführt und/oder das verwendete Medium hat eine Temperatur von 37 °C.

Gegenstand der Erfindung ist selbstverständlich auch das spezielle Zellmedium für die kurzzeitige Lagerung von, insbesondere nach Kryokonservierung aufgetauten, tierischen oder humanen Leberzellen insbesondere ein flüssiges Zellmedium, welches Natriumchlorid, Natriumglukonat, Natriumacetat, Kaliumchlorid, Magnesiumchlorid und Natriumcitrat, sowie Glucose und darüber hinaus Serumalbumin und/oder Blutplasma enthält, und worin die genannten Bestandteile in Wasser gelöst sind.

Ein bevorzugtes Zellmedium besteht aus Natriumchlorid, Natriumglukonat, Natriumacetat, Kaliumchlorid, Magnesiumchloridhexahydrat und Natriumcitrat, sowie Glucose und darüber hinaus Serumalbumin und/oder Blutplasma, wobei die genannten Bestandteile in Wasser gelöst sind. Ein besonders bevorzugtes Zellmedium enthält von 0,45 bis 0,60 Gew.-% Natriumchlorid, von 0,45 bis 0,55 Gew.-% Natriumglukonat, von 0,20 bis 0,25 Gew.-% Natriumacetat, von 0,03 bis 0,04 Gew.-% Kaliumchlorid; von 0,025 bis 0,035 Gew.-% Magnesiumchloridhexahydrat, und von 0,31 bis 0,33 Gew.-% Natriumcitrat, sowie Glucose und darüber hinaus Serumalbumin und/oder Blutplasma, wobei die genannten Bestandteile in Wasser gelöst werden.

Bevorzugt enthält das Zellmedium 0,01 bis 0,5 Gew-% Glucose, besonders bevorzugt von 0,1 bis 0,2 Gew-% Glucose.

Erfindungsgemäß bevorzugt enthält das Zellmedium Humanserumalbumin. Das Humanserumalbumin kann erfindungsgemäß durch humanes, insbesondere autologes, Blutplasma ersetzt werden. In einer bevorzugten Ausführungsform enthält das Medium von 0,5 bis 50 Gew-% Serumalbumin, besonders bevorzugt von 3 bis 5 Gew-% Serumalbumin. In einer anderen bevorzugten Ausführungsform enthält das Medium von 0,5 bis 50 Gew-% Blutplasma, besonders bevorzugt von 3 bis 5 Gew-% Blutplasma.

Das erfindungsgemäße Zellmedium hat vorzugsweise einen pH-Wert von 6,4 bis 8, bevorzugt von 7,0 bis 7,4, insbesondere von 7,2.

Erfindungsgemäß bevorzugt sind die Bestandteile eines erfindungsgemäßen Zellmediums gesetzlich für die Applikation, insbesondere die intravenöse Applikation, in einen tierischen Organismus, bevorzugt in einen menschlichen Organismus, zugelassen.

Erfindungsgemäß bevorzugt sind auch Kombinationen erfindungsgemäßer Ausführungsformen des erfindungsgemäßen Verfahrens und/oder des erfindungsgemäßen Zellmediums.

Die Erfindung umfasst auch die Verwendung des vorgenannten erfindungsgemäßen Zellmediums zur kurzzeitigen Lagerung von nach Kryokonservierung aufgetauten tierischen Leberzellen insbesondere nach dem erfindungsgemäßen Verfahren.

Die Figuren zeigen:
Figur 1: Vitalität der gelagerten Zellen (nach Kryokonservierung) in Abhängigkeit von der Lagerungszeit, dem verwendeten Lagerungsmedium und der Lagerungstemperatur,
   Legende: "HH142": Nummer der individuellen Zellpräparation (Charge), "warm":Lagerungstemperatur von 18 °C, "kalt": Lagerungstemperatur von 4 °C, "HES": Medium 2, "PBS": Medium 5, "Custodiol^{®}": Medium 1, "Composol^{®}": Medium 4, "RingerLactat": Medium 3, "humanes Plasma": Medium 6;
Figur 2: Wiederfindung der gelagerten Zellen (vor Kryokonservierung) in Abhängigkeit von der Lagerungszeit, dem verwendeten Lagerungsmedium und der Lagerungstemperatur, Legende siehe Figur 1;
Figur 3: Vitalität gelagerter Zellen (nach Kryokonservierung) in Abhängigkeit von der Lagerungszeit, dem verwendeten Lagerungsmedium und der Lagerungstemperatur, Legende siehe Figur 1;
Figur 4: Lebendzellzahl (LZZ) gelagerter Zellen (nach Kryokonservierung) in Abhängigkeit von der Lagerungszeit und dem verwendeten Lagerungsmedium, Legende siehe Figur 1;
Figur 5: Lebendzellzahl (LZZ) gelagerter Zellen (vor Kryokonservierung) in Abhängigkeit von der Lagerungszeit und dem verwendeten Lagerungsmedium, Median über drei individuelle Zellpräparationen (Chargen); Legende siehe Figur 1;
Figur 6: Lebendzellzahl (LZZ) gelagerter Zellen (vor Kryokonservierung) in Abhängigkeit von der Lagerungszeit und dem verwendeten Lagerungsmedium, Median über vier individuelle Zellpräparationen (Chargen); Legende siehe Figur 1;
Figur 7A-C: Wiederfindung, Lebendzellzahl (LZZ) gelagerter Zellen (vor Kryokonservierung) in Abhängigkeit von der Lagerungszeit und dem verwendeten Lagerungsmedium, Median über 3 individuelle Zellpräparationen (Chargen); Figur 7C: Vergleich mit Custodiol (=100%), Mittelwert über vier Lagenrungszeiten, Median über 3 individuelle Zellpräparationen (Chargen), Legende siehe Figur 1.

### Beispiel

Es wurden kryokonservierte Leberzellen aus speziellen Kryobeuteln/Kryoröhrchen verwendet. Da die Anzahl der vorhandenen Kryoröhrchen aus einer individuellen Zellpräparation für den Umfang der Studie nicht ausreichend waren, wurden sie nur zum Vergleich der Ergebnisse hinzugezogen. Alle Arbeitsschritte wurden unter aseptischen Bedingungen durchgeführt. Die Zellsuspension war vor Zugabe des Zellmediums vollständig aufgetaut. Das Auftauen der Kryoröhrchen erfolgte zeitlich unabhängig vom Auftauen der Beutel. Das Zellmedium wurde langsam zu den vorgelegten Zellen gegeben.

Um vergleichbare Ergebnisse zu erzielen, wurden die verschiedenen Versuche parallel von mindestens 2, gegebenenfalls 3 Personen durchgeführt.

Folgende Auftaumedien wurden hergestellt:
- Medium 1:: Custodiol^{®} (Vergleichsbeispiel)
- Medium 2:: Hydroxyethylstärke (HES) + 1 g/l Glucose + 4% HSA (Vergleichsbeispiel)
- Medium 3:: Ringer-Lactat + 1 g/l Glucose + 4% HSA (Vergleichsbeispiel)
- Medium 4:: Composol^{®} + 1 g/l Glucose + 4% HSA (erfindungsgemäß)
- Medium 5:: Phosphatgepufferte Saline (PBS) + 1 g/l Glucose + 4% HAS (Vergleichsbeispiel)
Medium 6: humanes Blutplasma (Vergleichsbeispiel)

Pro Durchlauf (ein aufgetauter Beutel) wurden ca. je 50 ml Zellmedium benötigt. Humanes Blutplasma wurde im 37 °C warmen Wasserbad aufgetaut.

Bei drei Versuchen wurden die Medien im 37 °C warmen Wasserbad für mindestens 15 min temperiert (= "warm"). Bei 2 Versuchen wurden die Zellmedien im Kühlschrank für mindestens 15 min. gekühlt (="kalt").

Sechs entsprechend den Lösungen etikettierte 50 ml-Röhrchen wurden vorgelegt und bei Raumtemperatur aufbewahrt.

Es wurde bei "warmen" Versuchen für 5 min bei 18 °C und 50 g zentrifugiert und bei "kalten" Versuchen für 5 Minuten bei 4 °C und 50 g zentrifugiert.

Kryobeutel wurden vorsichtig aus der Aluminiumkassette herausgenommen. Die Kryosuspension wurde 2 bis 5 min unter ständigem Schwenken aufgetaut bis keine Eiskristalle mehr zu sehen waren. Der gesamte Vorgang dauerte nicht länger als 5 min.

Die gelbe Kappe wurde vom Beutel entfernt und das Septum mit Anstechdorn eines Benjamix durchstochen. An den Benjamix des Kryobeutels wurde eine 60 ml Spritze angeschlossen und 60 ml Zellsuspension wurden entnommen.

Jeweils 10 ml Zellsuspension wurden in den sechs vorbereiteten Röhrchen überführt. Dazu wurden jeweils 40 ml von den entsprechenden Zellmedien langsam zugegeben. Der gesamte Vorgang dauerte nicht länger als 5 min.

Nach der Zentrifugation erfolgte das Waschen der Zellen. Es wurden die Überstände mit Pasteurpipette abgesaugt. Die Zellpellets wurden durch vorsichtiges Schwenken in 5 ml des entsprechenden Zellmediums vollständig resuspendiert.

Es erfolgte eine Zellzählung nach Zellzahl und Vitalität und nach jeder Zellzählung wurde eine Leberzell-Kultur angelegt.

Für eine Gesamtlagerdauer von 3 Stunden wurden jeweils nach 60 min die beiden letztgenannten Schritte wiederholt.

Es wurden nur Leberzellen mit einer Vitalität größer als 40% vor der Kryokonservierung verwendet. Der Versuch wurde mindestens 3 mal mit unterschiedlichen individuellen Zellpräparationen (Chargen) wiederholt. Dabei wurde pro Charge mindestens 1 Beutel aufgetaut. Zum Vergleich wurden die Ergebnisse vom Auftauen von Kryoröhrchen der verwendeten Chargen herangezogen.

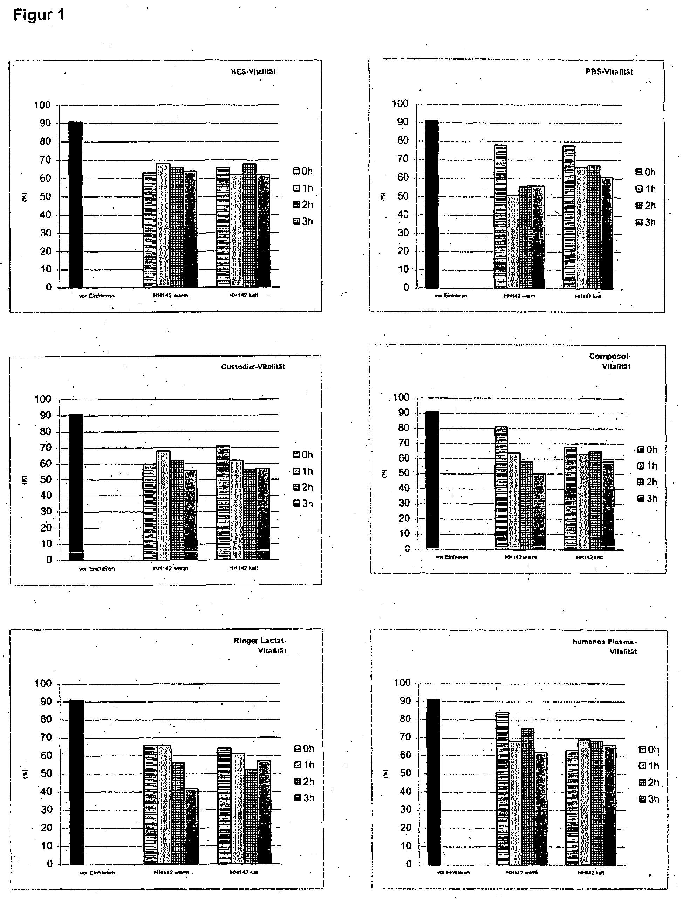

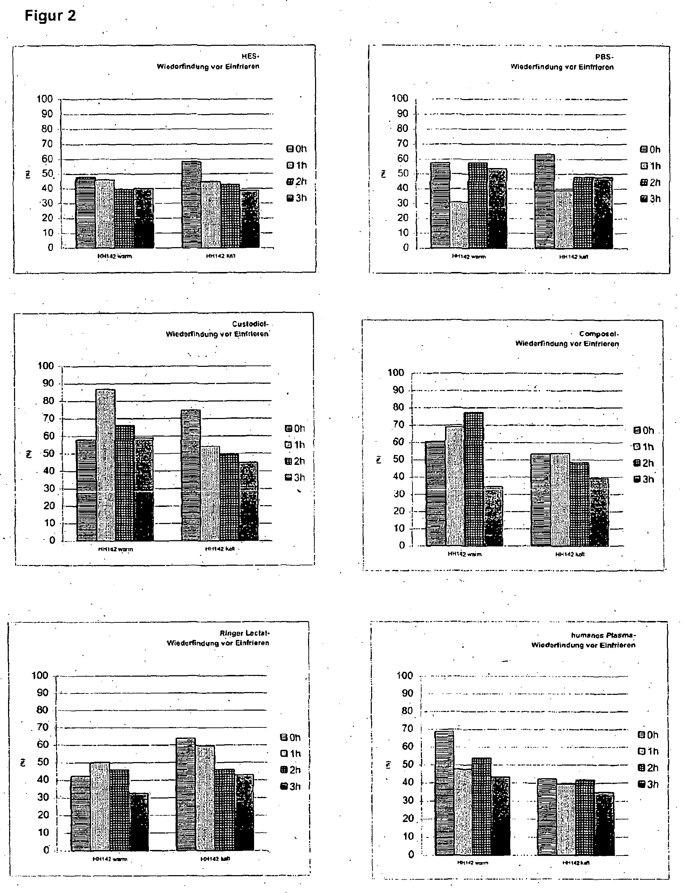

Es wurden vier individuelle Zellpräparationen (Chargen) hinsichtlich des Einflusses der Auftaulösung getestet. Die Ergebnisse wurden hinsichtlich der Parameter Vitalität und Lebendzellzahl (LZZ) nach Auftauen sowie Vitalitäts- und Lebendzellzahlerhalt bei Lagerung in Suspension getestet. Die Temperatur des Mediums und für die Zentrifugation wurde bei Leberzelltransplantaten auf 4 °C festgelegt. Der Versuch mit Charge HH142 wurde einmal "warm" durchgeführt und dann "kalt" wiederholt (siehe Figur 1 und Figur 2). Für die weitere Auswertung wurden die Mittelwerte von beiden Versuchen genommen und als HH142 bezeichnet.

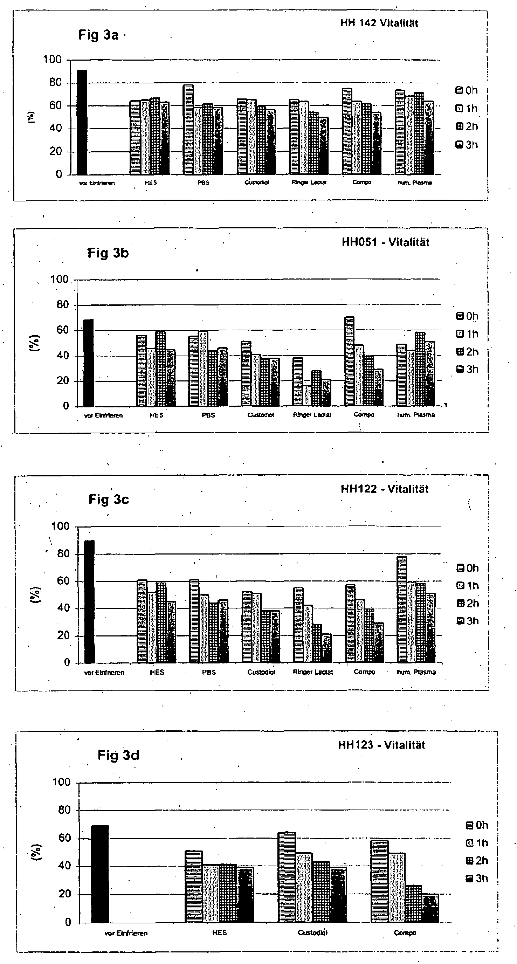

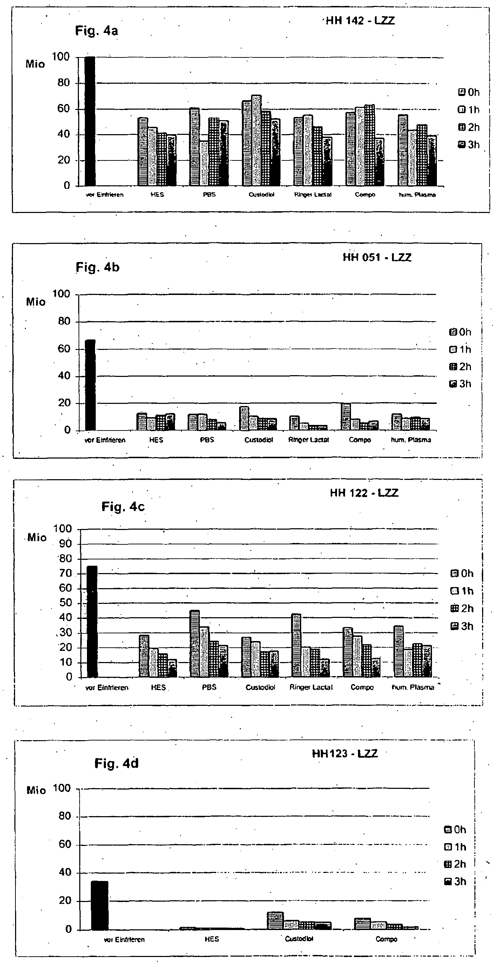

Die einzelnen Ergebnisse sind in den Figuren 3 und 4 dargestellt.

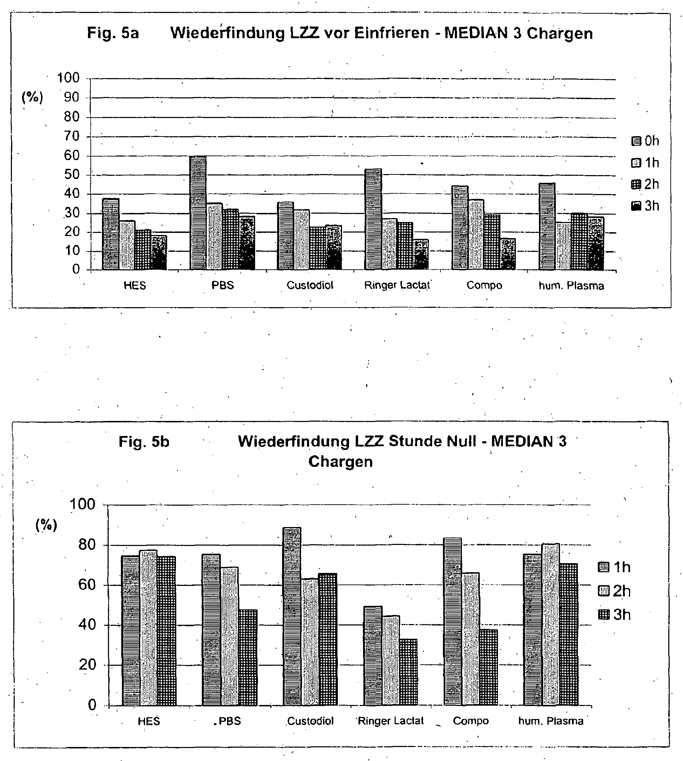

Um eine chargenunabhängige Auswertung zu gewährleisten, wurde der Parameter Wiederfindung der Lebendzellzahl bei der Lagerung in den getesteten Lösungen definiert. Dieser wurde auf zwei verschiedenen Ausgangspunkte bezogen. Zum einen die Wiederfindung bezogen auf die Lebendzellzahl vor Einfrieren (Figur 5a). Zum anderen die Wiederfindung bezogen auf die Lebendzellzahl nach Waschen (Stunde 0 in spezifischen Lagermedium) (Figur 5b). Zum Vergleich der fünf Lösungen wurden Medianwerte für die genannten Parameter aus den drei getesteten Chargen berechnet und in Figur 5 graphisch dargestellt.

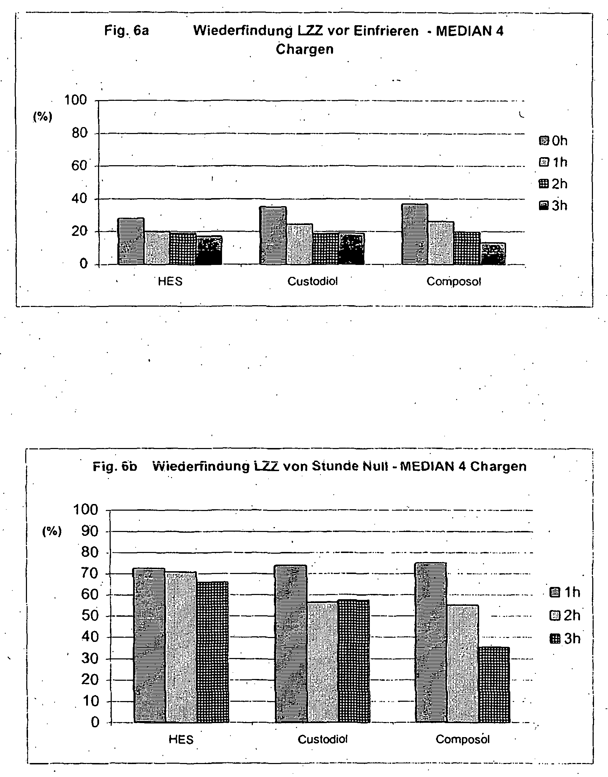

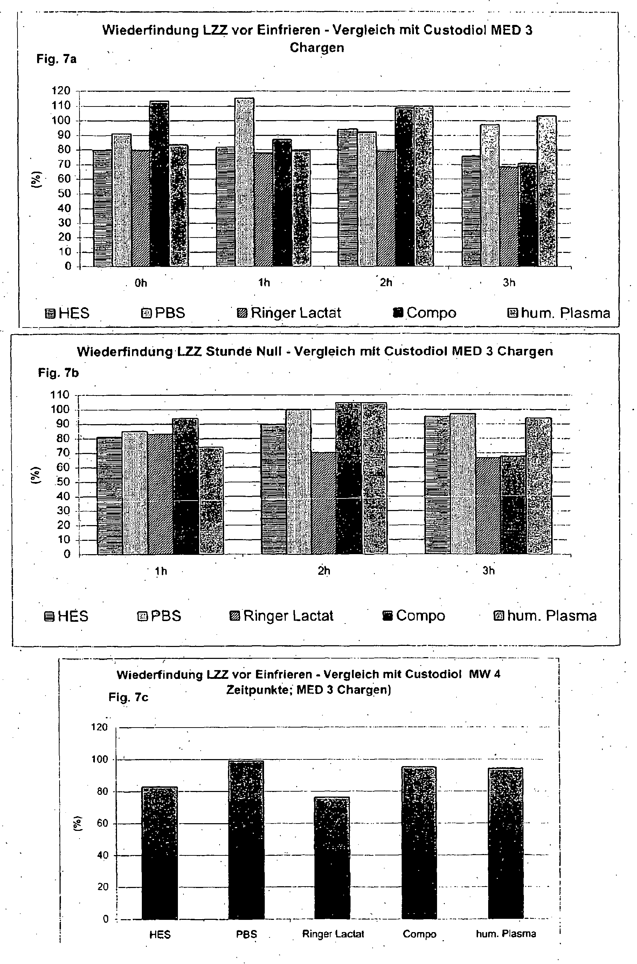

Die beiden ausgewählten Lösungen - Composol^{®} und HES -wurden zusätzlich anhand der Medianwerte von vier Chargen ausgewertet und in Figur 6 graphisch dargestellt:
Für eine bessere Analyse der dargestellten Ergebnisse wurde folgende Annährung gemacht. Der Parameter Wiederfindung der eingefrorenen LZZ wurde beim Custodiol^{®} 100% gesetzt. Für die getesteten fünf Ansätze (Vergleichsbeispiel/erfindungsgemäße Beispiele) wurde die prozentuale Abweichung dieses Parameters im Vergleich mit Custodiol^{®} berechnet (siehe Figur 7a und Figur 7b).

Direkt nach dem Waschen der Zellen zeigten drei der Lösungen (PBS, Composol^{®} und HES) eine vergleichbare (Übereinstimmung größer 80%) mit Custodiol^{®} hinsichtlich des Parameters Wiederfindung der LZZ. Dabei zeigte Composol^{®} sogar bessere Ergebnisse als Custodiol^{®} (Wiederfindung größer 100%). Während nach 1 h PBS die besten Ergebnisse aufwies zeigten Composol^{®} und humanes Plasma nach 2 Stunden wiederum etwa 10% bessere mediane Ergebnisse als Custodiol^{®}.

Zum weiteren Vergleich wurde eine weitere Auswertung durchgeführt. Für jede Lösung wurde der Mittelwert aus allen 4 Zeitpunkten berechnet (Stunde 0 bis 3, Figur 7c).

Unabhängig von Zeitvorlauf des Versuchs zeigten HES und Ringer-Lactat ca. 20% Verlust der LZZ im Vergleich mit Custodiol^{®}, wobei PBS, Composol^{®} und humanes Plasma vergleichbare (Abweichung kleiner 10%) Ergebnisse wie Custodiol^{®} zeigten.

## Patentansprüche

1. Verfahren zur kurzzeitigen Lagerung von Leberzellen, wobei die Zellen in einem flüssigen Zellmedium enthaltend
a) Salzlösung,
- enthaltend Natriumchlorid, Natriumglukonat, Natriumacetat, Kaliumchlorid, Magnesiumchlorid und Natriumcitrat,
b) Glucose und
c) Serumalbumin und/oder Blutplasma
aufgenommen werden und in diesem Zellmedium gelagert werden.

2. Verfahren nach Anspruch 1, wobei die Zellen kryokonservierte Zellen sind und aufgetaut werden und nach dem Auftauen in dem Zellmedium gewaschen werden, vom Zellmedium abgetrennt werden und dann in frischem Zellmedium zur Lagerung aufgenommen werden.

3. Verfahren nach einem der vorhergehenden Ansprüche, wobei das Zellmedium eine Temperatur von 2 bis 40 °C hat.

4. Verfahren nach einem der vorhergehenden Ansprüche, wobei es sich bei den Zellen um Hepatozyten handelt.

5. Verfahren nach einem der vorhergehenden Ansprüche, wobei es sich bei den Zellen um Säugetier-Zellen handelt.

6. Verfahren nach einem der vorhergehenden Ansprüche, wobei es sich bei den Zellen um humane Zellen handelt.

7. Verfahren nach einem der vorhergehenden Ansprüche, wobei es sich bei den Zellen um Zellen zur therapeutischen Applikation handelt.

8. Verfahren nach einem der Ansprüche 1 bis 6, wobei es sich um Zellen zur *in vitro* Kultivierung handelt.

9. Verwendung eines Zellmediums wie in Anspruch 1 charakterisiert zur kurzzeitigen Lagerung von Leberzellen, insbesondere nach dem Verfahren nach einem der Ansprüche 1 bis 8.

10. Zellmedium für die kurzzeitige Lagerung von Leberzellen, wobei das Zellmedium
a) Natriumchlorid, Natriumglukonat, Natriumacetat, Kaliumchlorid, Magnesiumchlorid und Natriumcitrat,
b) Glucose und
c) Serumalbumin und/oder Blutplasma
in Wasser gelöst enthält.

11. Zellmedium nach Anspruch 10, wobei das Serumalbumin Humanserumalbumin ist.

12. Zellmedium nach einem der Ansprüche 10 oder 11, wobei das Blutplasma humanes Blutplasma ist.

13. Zellmedium nach einem der Ansprüche 10 bis 12, wobei das Zellmedium
von 0,45 bis 0,60 Gew.-% Natriumchlorid,
von 0,45 bis 0,55 Gew.-% Natriumglukonat,
von 0,20 bis 0,25 Gew.-% Natriumacetat,
von 0,03 bis 0,04 Gew.-% Kaliumchlorid,
von 0,025 bis 0,035 Gew.-% Magnesiumchloridhexahydrat,
und von 0,31 bis 0,33 Gew.-% Natriumcitrat enthält.

14. Zellmedium nach einem der Ansprüche 10 bis 13, wobei das Zellmedium von 0,01 bis 0,5 Gew.-%, bevorzugt 0,1 bis 0,2 Gew.-%, Glucose enthält.

15. Zellmedium nach einem der Ansprüche 10 bis 14, wobei das Zellmedium von 0,5 bis 50 Gew.-%, bevorzugt 3 bis 5 Gew.-%, Serumalbumin enthält.

16. Zellmedium nach einem der Ansprüche 10 bis 15, wobei das Zellmedium von 0,5 bis 50 Gew.-%, bevorzugt 3 bis 5 Gew.-%, Blutplasma enthält.

17. Zellmedium nach einem der Ansprüche 10 bis 16, wobei das Zellmedium einen pH-Wert von 6,4 bis 8, bevorzugt von 7,0 bis 7,4, hat.

18. Zellmedium nach einem der Ansprüche 10 bis 17, enthaltend mindestens einen weiteren Bestandteil, ausgewählt aus: Aminosäuren, Hormonen, Vitaminen, Provitaminen und antiapoptotisch wirksamen Substanzen

## Claims

1. Method for short-term storage of liver cells, wherein the cells are placed in a liquid cell medium containing
a) a salt solution,
- containing sodium chloride, sodium gluconate, sodium acetate, potassium chloride, magnesium chloride and sodium citrate,
b) glucose and
c) serum albumin and/or blood plasma
and are stored in this cell medium.

2. Method according to claim 1, wherein the cells are cryopreserved cells and are thawed and then after thawing are washed in the cell medium, separated from the cell medium and then placed in fresh cell medium for storage.

3. Method according to any one of the preceding claims, wherein the cell medium has a temperature of 2 to 40°C.

4. Method according to any one of the preceding claims, wherein the cells are hepatocytes.

5. Method according to any one of the preceding claims, wherein the cells are mammalian cells.

6. Method according to any one of the preceding claims, wherein the cells are human cells.

7. Method according to any one of the preceding claims, wherein the cells are cells for therapeutic administration.

8. Method according to any one of claims 1 through 6, wherein the cells are for in vitro culturing.

9. Use of a cell medium as **characterized in** claim 1 for short-term storage of liver cells, in particular according to the method according to any one of claims 1 through 8.

10. Cell medium for short-term storage of liver cells, wherein the cell medium contains
a) sodium chloride, sodium gluconate, sodium acetate, potassium chloride, magnesium chloride and sodium citrate,
b) glucose and
c) serum albumin and/or blood plasma
dissolved in water.

11. Cell medium according to claim 10, wherein the serum albumin is human serum albumin.

12. Cell medium according to any one of claims 10 or 11, wherein the blood plasma is human blood plasma.

13. Cell medium according to any one of claims 10 through 12, wherein the cell medium contains
0.45 to 0.60 wt% sodium chloride,
0.45 to 0.55 wt% sodium gluconate,
0.20 to 0.25 wt% sodium acetate,
0.03 to 0.04 wt% potassium chloride,
0.025 to 0.035 wt% magnesium chloride hexahydrate,
and 0.31 to 0.33 wt% sodium citrate.

14. Cell medium according to any one of claims 10 through 13, wherein the cell medium contains 0.01 to 0.5 wt%, preferably 0.1 to 0.2 wt% glucose.

15. Cell medium according to any one of claims 10 through 14, wherein the cell medium contains 0.5 to 50 wt%, preferably 3 to 5 wt% serum albumin.

16. Cell medium according to any one of claims 10 through 15, wherein the cell medium contains 0.5 to 50 wt%, preferably 3 to 5 wt% blood plasma.

17. Cell medium according to any one of claims 10 through 16, wherein the cell medium has a pH of 6.4 to 8, preferably 7.0 to 7.4.

18. Cell medium according to any one of claims 10 through 17 containing at least one additional ingredient selected from amino acids, hormones, vitamins, provitamins and antiapoptotic substance.

## Revendications

1. Procédé pour le stockage de courte durée des cellules hépatiques, dans lequel les cellules sont mises en suspension dans un milieu de culture cellulaire liquide contenant
a) de la solution saline,
- contenant du chlorure de sodium, gluconate de sodium, acétate de sodium, chlorure de potassium, chlorure de magnésium et citrate de sodium,
b) de la glucose et
c) de l'albumine de sérum et/ou plasma sanguin
et stockées dans ce milieu de culture cellulaire.

2. Procédé selon la revendication 1, dans lequel les cellules sont des cellules cryoconservées, et sont décongelées et lavées dans le milieu de culture cellulaire après la décongélation, sont séparées du milieu de culture cellulaire et puis remises en suspension dans du milieu de culture cellulaire frais pour le stockage.

3. Procédé selon l'une quelconque des revendications précédentes, dans lequel le milieu de culture cellulaire présente une température de 2 à 40 °C.

4. Procédé selon l'une quelconque des revendications précédentes, dans lequel les cellules sont des hépatocytes.

5. Procédé selon l'une quelconque des revendications précédentes, dans lequel les cellules sont des cellules de mammifère.

6. Procédé selon l'une quelconque des revendications précédentes, dans lequel les cellules sont des cellules humaines.

7. Procédé selon l'une quelconque des revendications précédentes, dans lequel les cellules sont des cellules pour l'application thérapeutique.

8. Procédé selon l'une quelconque des revendications 1 à 6, dans lequel les cellules sont des cellules pour la culture in vitro.

9. Utilisation d'un milieu de culture cellulaire tel que défini dans la revendication 1, caractérisé pour le stockage de courte durée des cellules hépatiques, notamment selon le procédé selon l'une quelconque des revendications 1 à 8.

10. Milieu de culture cellulaire pour le stockage de courte durée des cellules hépatiques, le milieu de culture cellulaire contenant
a) du chlorure de sodium, gluconate de sodium, acétate de sodium, chlorure de potassium, chlorure de magnésium et citrate de sodium,
b) de la glucose et
c) de l'albumine de sérum et/ou plasma sanguin
dissous dans de l'eau.

11. Milieu de culture cellulaire selon la revendication 10, dans lequel l'albumine de sérum est de l'albumine de sérum humaine.

12. Milieu de culture cellulaire selon l'une quelconque des revendications 10 ou 11, dans lequel le plasma sanguin est du plasma sanguin humain.

13. Milieu de culture cellulaire selon l'une quelconque des revendications 10 à 12, le milieu de culture contenant
de 0,45 à 0,60 % en poids du chlorure de sodium,
de 0,45 à 0,55 % en poids du gluconate de sodium,
de 0,20 à 0,25 % en poids de l'acétate de sodium,
de 0,03 à 0,04 % en poids du chlorure de potassium,
de 0,025 à 0,035 % en poids du chlorure de magnésium hexahydraté,
et de 0,31 à 0,33 % en poids de citrate de sodium.

14. Milieu de culture cellulaire selon l'une quelconque des revendications 10 à 13, le milieu de culture cellulaire contenant de 0,01 à 0,5 % en poids, de préférence de 0,1 à 0,2 % en poids, du glucose.

15. Milieu de culture cellulaire selon l'une quelconque des revendications 10 à 14, le milieu de culture cellulaire contenant de 0,5 à 50 % en poids, de préférence de 3 à 5 % en poids, de l'albumine de sérum.

16. Milieu de culture cellulaire selon l'une quelconque des revendications 10 à 15, le milieu de culture cellulaire contenant de 0,5 à 50 % en poids, de préférence de 3 à 5 % en poids, du plasma sanguin.

17. Milieu de culture cellulaire selon l'une quelconque des revendications 10 à 16, le milieu de culture cellulaire présentant une valeur pH de 6,4 à 8, de préférence de 7,0 à 7,4.

18. Milieu de culture cellulaire selon l'une quelconque des revendications 10 à 17, contenant au moins un autre ingrédient, choisi parmi : les acides aminés, les hormones, les vitamines, les provitamines et les substances à effet antiapoptotique.
